(19) Europäisches Patentamt

European Patent Office

Office européen des brevets

(11) **EP 4 075 446 B1**

(12) **EUROPEAN PATENT SPECIFICATION**

(45) Date of publication and mention
of the grant of the patent:
**03.09.2025 Bulletin 2025/36**

(21) Application number: **21169042.5**

(22) Date of filing: **18.04.2021**

(51) International Patent Classification (IPC):
***G16H 50/50*** *(2018.01)*

(52) Cooperative Patent Classification (CPC):
**G16H 50/50**

(54) **METHOD AND SYSTEM FOR MODELLING BLOOD VESSELS AND BLOOD FLOW UNDER HIGH-INTENSITY PHYSICAL EXERCISE CONDITIONS**

VERFAHREN UND SYSTEM ZUR MODELLIERUNG VON BLUTGEFÄSSEN UND BLUTFLUSS UNTER HOCHINTENSIVEN KÖRPERLICHEN ÜBUNGSBEDINGUNGEN

PROCÉDÉ ET SYSTÈME DE MODÉLISATION DES VAISSEAUX SANGUINS ET DU FLUX SANGUIN DANS DES CONDITIONS D'EXERCICE PHYSIQUE DE HAUTE INTENSITÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**19.10.2022 Bulletin 2022/42**

(73) Proprietor: **Kardiolytics Inc.**
**Tulsa 74114 (US)**

(72) Inventors:
• **MALOTA, Zbigniew**
**Tychy (PL)**

• **SADOWSKI, Wojciech**
**Zabrze (PL)**
• **SIEMIONOW, Kris**
**Chicago (US)**
• **LEWICKI, Paul**
**Tulsa (US)**

(74) Representative: **Kancelaria Eupatent.pl Sp. z o.o.**
**ul. Kilinskiego 185**
**90-348 Lodz (PL)**

(56) References cited:
**EP-A1- 3 629 341     US-A1- 2022 338 932**
**US-B1- 9 087 147**

**Description**

TECHNICAL FIELD

The present disclosure relates to modelling blood vessels and blood flow in the modeled blood vessels, in particular under high-intensity physical exercise conditions. The invention is applicable as a tool for medical practitioners, and to assist in obtaining information about the cardiovascular of a patient, which can be useful for further diagnosis of circulatory and cardiovascular diseases. The method described here allows to shorten the time of modeling blood flow through the blood vessels and thus speed up the obtaining the information about patient-specific blood circulation system.

BACKGROUND

Cardiovascular diseases are a serious problem, both in a health and a societal aspect. Atherosclerosis is a disease caused by lipoproteins, which leads to the formation of an atherosclerotic plaque at specific sites of an artery due to intramuscular inflammation, necrosis, fibrosis, and calcification. Stable forms of coronary heart disease can be often treated pharmacologically. In many cases, however, revascularization is necessary by means of either percutaneous angioplasty or through coronary artery bypass grafting (CABG), which redirects blood around a section of a blocked or partially blocked artery.

In order to determine the course of a disease and to select which therapeutic actions should be taken, it is necessary to conduct diagnostic tests. Currently, the basic methods for assessing the hemodynamic significance of coronary stenosis are cardiac stress tests, contrast angiography, and fractional flow reserve (FFR). Practically all methods for assessing the significance of hemodynamic coronary stenosis have some disadvantages.

The main requirement of the FFR is that maximal dilation of downstream resistance vessels be achieved pharmacologically. The Crystal George J. in "Fractional Flow Reserve: Physiological Basis, Advantages and Limitations and Potential Gender Differences". Current Cardiology Reviews, 11, 209-219. 2015, claim that the use of FFR requires an in-depth knowledge of the technique, including its limitations and sources of variability, an appreciation of the rheological and physical properties of coronary stenoses, and an understanding of the physiology, pathophysiology, and pharmacology of the coronary microvasculature.

The exercise stress test allows assessment of the heart's function in response to increased physical effort and increased oxygen demand. It is a stress test and therefore, there are numerous contraindications to its performance, such as: very advanced ischemic heart disease, advanced aortic valve disease, severe heart failure, acute infection, recent stroke, unstabilized arterial hypertension and osteoarthritis.

In turn, contrast angiography, despite being a high spatial resolution test, has limitations related to image analysis and the invasive nature of the procedure. One of the limitations is the fact that visualization of the cross-sectional area of the vessel does not provide any information about the arterial wall. Another limitation is that the three-dimensional image demonstrating the course of arteries, mapped on two-dimensional plane, causes that on various images eccentric atherosclerotic plaques are projected as changes having variable degree of stenosis.

Fractional flow reserve measurement is also an invasive technique, performed during angiography, to measure pressure differences across a region of coronary artery stenosis. However, a non-linear relationship between pressure-flow, as well as the influence of external vessel forces, size of the perfusion area, microvascular dysfunction, as well as catheter-induced errors in invasive measurements of pressure can affect the accuracy of FFR assessment. The use of FFR requires a deep knowledge of the anatomy, physiology, and pathophysiology of coronary arteries. The degree of narrowing does not transfer easily into the functional significance of the change. The decision about revascularization in relation to intermediate stenosis, when reduction in the diameter of the artery lumen is in the range from 50% to 70%, is particularly controversial. It is estimated that about half of them are hemodynamically significant, although they are often overlooked at revascularization.

Inconveniences and risks for patients associated with these tests are widely known and led to the development of supportive methods of obtaining information about the cardiovascular system, the results of which are intended for medical practitioners and can be used to assess changes occurring in cardiovascular diseases.

EP2845537 discloses a method for modelling blood flow in which a three-dimensional model comprising at least a part of the patient's heart is computed based on medical imaging and physiological data. Computing the model requires a series of non-invasive measurements carried out in the patient, for example performing a coronary angiography, measuring the stroke volume, blood pressure, etc. Based on that, a three-dimensional model representing the patient's heart geometry is defined, but also the boundary and initial conditions necessary for further modelling of blood flow are defined, which based on the mentioned tests, are determined individually for each patient. The model computed on the basis of such data, maps the anatomic features of the patient's heart. In the next step, the computed model is divided into segments and the blood flow to individual segments or between individual segments is determined, based on the boundary conditions determined at an earlier step. The modelled blood flow can take into account different states of the patient's heart, for example, mapping the state for the resting heart rate or the pulse achieved during the maximum exercise. Flow modelling based on boundary conditions also allows to induce different phases of the patient's heartbeat. Flow rate modelling allows reading various parameters related to the heart and blood flow, visualizing this flow, and also allows to spot changes in the circulatory system.

US2014249790A1 discloses a system and a method for assessing blood circulatory treatment in a patient. The method includes computing a three-dimensional model representing a part of a patient's heart based on patient-specific data relating to a patient's heart geometry or a vascular system. Suitable models are computed for many options for treatment of the patient's heart or the vascular system by modifying at least one three-dimensional model and a 2D-reduced model that is based on a the three-dimensional model. The computed models may include determining a line of the central course of blood vessels, which can also be modified and verified on the basis of available medical tests. The method also includes determining, for each of the plurality of treatment options, blood flow characteristics, by solving flow equations in at least one of the models - in a the modified three-dimensional model or the modified model of reduced dimensionality with the identification of treatment options.

US2017265831A1 also discloses a method for estimating diameter of a healthy coronary artery and assessing stenoses in these coronary arteries. This method firstly conducts medical examinations of many subjects, gathering information on diameters and other characteristics of healthy blood vessels. In the next step, the patient is examined and the diameter of his blood vessels is determined based on these tests. In the next stage, the diameter of healthy blood vessels is estimated based on the previous tests carried out among other subjects. Only then the degree of stenosis of the diameter of blood vessels is calculated based on such collected data. Essentially, this parameter is calculated as the ratio of the diameter of the blood vessels of the patient to be examined and the diameters of the theoretically healthy blood vessels estimated from other subjects. The degree of stenosis of blood vessel diameters is calculated for selected segments of blood vessels, wherein comparing two models of blood vessels can also take into account other features of blood vessels, such as their length.

US2014200867 discloses a method for vascular assessment that comprises receiving a plurality of 2D angiographic images of a portion of a vasculature of a subject, and processing the images to produce a stenotic model over the vasculature, the stenotic model having measurements of the vasculature at one or more locations along vessels of the vasculature. The method further comprises obtaining a flow characteristic of the stenotic model, and calculating an index indicative of vascular function, based, at least in part, on the flow characteristic in the stenotic model.

US2015065846 discloses a method for predicting the location, onset, or change of coronary lesions from factors like vessel geometry, physiology, and hemodynamics. One method includes: acquiring, for each of a plurality of individuals, a geometric model, blood flow characteristics, and plaque information for part of the individual's vascular system; training a machine learning algorithm based on the geometric models and blood flow characteristics for each of the plurality of individuals, and features predictive of the presence of plaque within the geometric models and blood flow characteristics of the plurality of individuals; acquiring, for a patient, a geometric model and blood flow characteristics for part of the patient's vascular system; and executing the machine learning algorithm on the patient's geometric model and blood flow characteristics to determine, based on the predictive features, plaque information of the patient for at least one point in the patient's geometric model.

US2012041318 discloses a system for determining cardiovascular information for a patient. The system may include at least one computer system configured to receive patient-specific data regarding a geometry of the patient's heart, and create a three-dimensional model representing at least a portion of the patient's heart based on the patient-specific data. The at least one computer system may be further configured to create a physics-based model relating to a blood flow characteristic of the patient's heart and determine a fractional flow reserve within the patient's heart based on the three-dimensional model and the physics-based model.

EP3629341A1 discloses a method for modelling blood vessels, the method comprising the steps of: obtaining medical imaging data of the blood vessels; generating a three-dimensional personalized model of the blood vessels, based on the medical imaging data; generating a three-dimensional reconstructed model (i.e. the reference model), of the blood vessels that reflects a state of healthy blood vessels that lack lesions, based on the medical imaging data or based on numerical reconstruction of the personalized model; performing a numerical simulation of blood flow for the same physical and boundary conditions in the personalized model and in the reconstructed model, the simulation comprising determining conditions of blood flow at an inlet to the blood vessels model and calculating blood flow energy for the inlet and all outlets of the blood vessels model; comparing the blood flow energy measured for the personalized model and for the reconstructed model; determining flow energy change indexes of the blood flow in the personalized model and in the reconstructed model.

Inconveniences and risks for patients associated with these tests are widely known and led to the development of supportive methods of obtaining information about the cardiovascular system, the results of which are intended for medical practitioners and can be used to assess changes occurring in cardiovascular diseases.

US 9087147 B1 discloses a method for determining boundary conditions of a blood vessel model in case of a steady flow of blood and a transient flow of blood. This document is however silent about the specific mathematical modelling herein described.

## SUMMARY OF THE INVENTION

The purpose of this invention is to solve problems that occur in known methods of supplementing information on cardiovascular diseases, the results of which are intended for medical practitioners and which can be used in further diagnostics. Essentially, the analysis of blood flow dynamics in generated models reflecting blood vessels itself is very difficult mainly due to complex geometry of the blood vessels, the flexibility of their walls, non-Newtonian properties of blood, pulsatile flow and variable vascular resistance, during the diastolic and systolic phases of the cardiac cycle. Particular difficulties are related to both the lack of detailed clinical data as well as individual (personalized) differences in cardiovascular physiology enabling precise mathematical and physical models generation.

Certain embodiments of this invention allow shortening the time of modeling the cardiovascular system as compared with the prior art methods discussed above.

The use of 3D models carry several challenges, which range from 3D segmentation of coronary arteries and mesh generation to time-consuming numerical simulations.

In turn, simplified mathematical models, zero-dimensional and one dimensional models, based on simplified representations of the components of the cardiovascular system, neglect fundamental physical properties of the 3D flow across variable geometry (arch, bifurcation, stenosis). Additionally, these complex lumped models require the definition of many patient specific parameters describing peripheral resistance and blood vessel compliance. The proper development of a numerical model of coronary vessels, that would be completely consistent with clinically obtained data, is a great challenge for researchers and very often requires invasive measurements.

An advantage of the present invention is that it allows for modelling of blood vessels and blood flow in these blood vessel models based only on non-invasive medical imaging data, such as computed tomography.

The method comprises conducting blood flow simulation for a personalized model and for a reconstructed model of blood vessels.

Certain embodiments of the present invention make use of the physical properties (pressure relationships) occurring during the increasing inlet blood flow rate, from laminar flow until achieving a developed turbulent flow.

Additionally, increased physical exertion can elicit cardiovascular abnormalities that are not present at rest, and it can be used to determine the stage of coronary heart disease.

Heart rate and systolic blood pressure linearly increases with increasing work load (as discussed by Fletcher GF et al, in "Exercise Standards for Testing and Training. A Statement for Healthcare Professionals From the American Heart Association". Circulation. 104:1694-1740. 2001). This increase is dependent on age, LV dysfunction, muscle ischemia, and peripheral resistance (as discussed by Wolthuis RA et al, in "The response of healthy men to treadmill exercise". Circulation. 55: 153-157. 1977). Normal systolic responses to exercise stress testing should not exceed 220 mmHg (30 kPa), while normal values for heart rate responses to exercise reach 200 cycles per minute.

However, some embodiments of present inventions does not require variable boundary conditions. In some embodiments, the method of the invention allows the use of a steady blood flow under fixed value of inlet pressure and outlets flow rate conditions.

For flow through tree coronary vessels, there is a global pressure loss over the vessel length due to friction and local pressure loss due change of geometries, directly at the site of vessel stenoses, branches or bends. An increase in flow rate, during a virtual exercise test, causes an increase in pressure drop, which depends on the geometry of the coronary vessels, the degree of stenosis, its shape and place position and hemodynamic conditions prevailing in the cardiovascular system (as discussed by Malota Z. et al in "Numerical analysis of the impact of flow rate, heart rate, vessel geometry, and degree of stenosis on coronary hemodynamic indices" BMC Cardiovascular Disorders. 18:132. 2018).

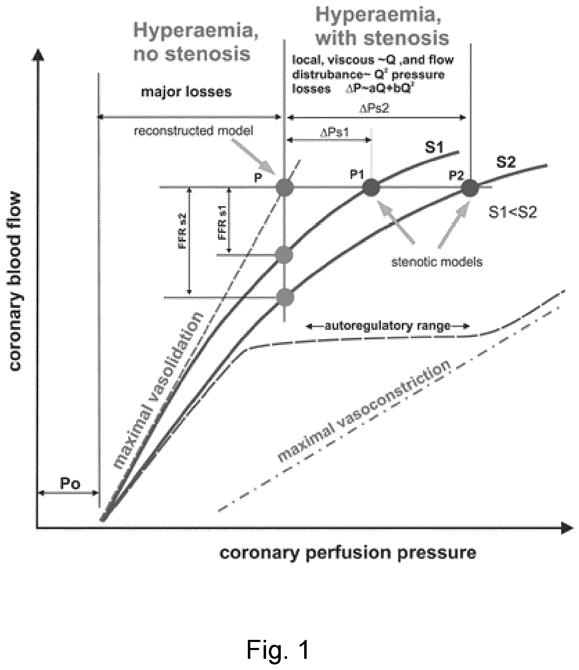

In the model with stenosis (i.e. the personalized model), this increase is much higher than in the reconstructed model (i.e. the reference model), see Fig.1.

In the case of vessels with stenosis, the drop in pressure in a narrowed vessel can be represented in a simplified way as a function of pressure gradient and flow rate. The function is a quadratic polynomial where the first element determines the effect of the viscosity forces and the second one is due to the convective acceleration and inertial forces, which lead to energy losses through flow separation, flow disturbance and turbulence flow (Young DF, Tsai FY. (1973). "Flow characteristics in models of arterial stenoses: I Steady flow". J Biomech.6:395-410).

The principles of the test are shown on the graph of coronary perfusion pressure-flow relationship in the presence of stenosis from rest conditions to high physical exertion conditions (Fig.1), adapted on the basis of work Duncker D. et al (2015) ("Regulation of Coronary Blood Flow in Health and Ischemic Heart Disease". Prog Cardiovasc Dis. 57(5):409-422) and Casadonte L., Siebes M. (2017) ("Hemodynamic Effects of Epicardial Stenoses". In: Escaned J., Davies J. (eds) Physiological Assessment of Coronary Stenoses and the Microcirculation. Springer, London).

Due to the regulation of coronary vascular resistance (vasodilation) blood flow is maintained relatively constant over a wide range of perfusion pressures (Fig.1). Outside this range, flow become pressure-dependent and the loss of autoregulation is observed. The S1 curve represents a stenotic vessel with a lesser degree of narrowing compared to S2, under maximum vasodilation conditions (solid lines). The point P reflect the hypothetical state of patient healthy vessels without stenosis. Points P1 and P2 reflect the actual state of coronary vessels of the patient with existing atherosclerotic lesion. For normal coronary vessel, without stenosis, under maximal vasodilation (dashed line), the loss of flow energy is associated mainly with viscous forces.

The invention is based on the three main assumptions:

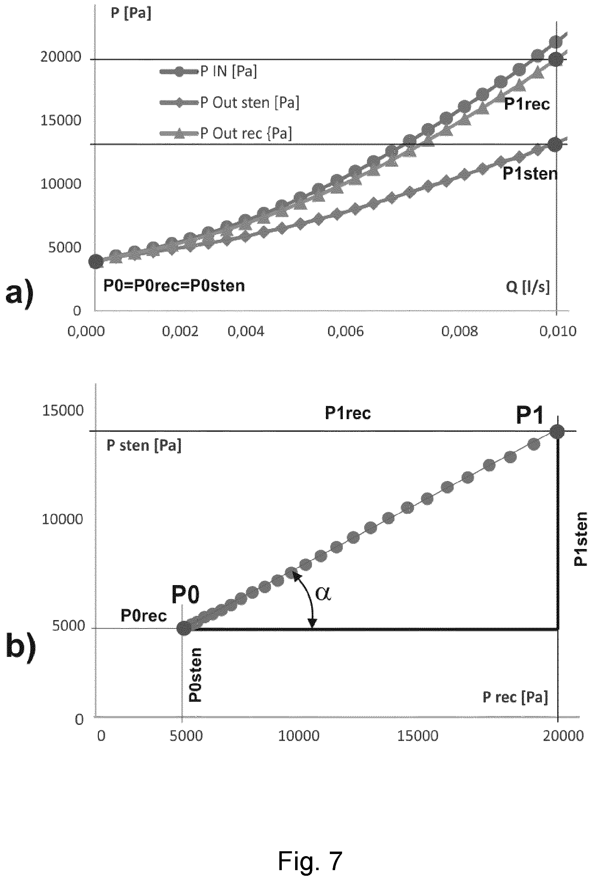

- comparative method that makes possible to determine the relationship between pressure or velocity in the model with stenosis and the pressure in the reconstructed model under the same hemodynamic conditions,
- simulation under maximum vasodilation conditions when auto-regulation effect still had the ability to maintain the flow rate with minimal peripheral resistance, which is characterized by high blood flow causing an increase in flow disturbances and thus an increase in energy loss during the flow through the stenosis,
- high linear correlation, as research by the inventors showed, see Fig.7, between distal pressure, downstream to the stenosis and the pressure in the same place in the reconstructed model, during linear increase of flow rate. The curve slope of this relationship can be used to determine the hemodynamic significance of vessel stenosis.

The use of the comparative method, where relative indexes are determined based on a comparison of two models, the personalized model (with stenosis) and the reconstructed model, of the same patient makes it possible to significantly limit the influence of errors on the final result of the test and first of all it allows on determinate this indexes based on steady flow under high exercise condition.

According to a publication by Lo et al in "On outflow boundary conditions for CT-based computation of FFR: Examination using PET images", Med Eng Phys. 76:79-87. doi: 10.1016/j.medengphy.2019.10.007. 2020), there is high correlation between flow reserve indexes values obtained using a pulsatile simulation and that of a steady-state simulation. Based on the findings of this work, the maximum discrepancy in numerical computed flow reserve indexes from steady and unsteady flow simulations is around 2%.

Therefore, finally, some indexes that discloses in EP3629341A1, such as $FFR_{VCAST}$, the reference fractional flow reserve index, and EFR, the energy flow reference index, can be calculated for steady flow under fixed value of flow rate conditions, that significantly reduce the numerical computation time.

Using numerical calculations for steady flow instead calculations for transient flow shortens the time needed to obtain test results from several hours to several minutes. It is extremely important from the point of view of the diagnosis of coronary vessels and the quick selection of the treatment procedure.

The determine of parameters, mentioned above, for the steady-flow, at any point in this curve, it means under fixed value of flow rate conditions, is possible due to the constant slope of curve relationship between distal pressure, downstream to the stenosis and the pressure in the same place in the reconstructed model (Fig.7).

The method as presented herein allows to simulate blood flow, in models of blood vessels, under the same physical and boundary conditions, when the blood flow conditions at the inlet and/or outlet to the blood vessel models are regulated, and the flow energy is measured in all branches of these models. The flow distribution and inlet pressure condition in the personalized model of the patient is simulated for the boundary conditions obtained from the results of flow simulations in the reconstructed model.

Blood flow in the blood vessels can be increased to values greater from typical rest physiological values through increased physical effort.

Increased physical exertion, increases the flow rate and can elicit coronary flow disturbances that do not occur at rest. The flow becomes transient or turbulent.

The higher the flow rate, the higher the energy drop induced by stenosis, the more accurate assessment of the significance of the stenosis.

The method allows the medical practitioner that analyzes the results of the blood flow simulation in the blood vessel models, to obtain additional diagnostic information that may be useful to assess the nature and significance of changes occurring in the examined cardiovascular model without the necessity for additional tests of the patient

In contrast to other non-invasive tests of evaluation of coronary stenosis significance, the compare test performed for high steady flow rate under maximal vasodilation conditions is much faster.

It is extremely important from the point of view of the diagnostics of coronary vessels and selection of the treatment procedure. The reduction of time diagnosis can reduce risks, of complications and thus, improving the results of long-term treatment of patients.

The object of the invention is a computer-implemented method for modelling blood vessels, the method comprising the steps of: obtaining medical imaging data of the blood vessels; generating a three-dimensional personalized model of the blood vessels, based on the medical imaging data; generating a three-dimensional reconstructed model of the blood vessels that reflects a state of healthy blood vessels that lack lesions, based on the medical imaging data or based on numerical reconstruction of the personalized model; performing pre-simulation of reconstructed model, establishing boundary conditions and initial conditions for both models for a steady flow of blood and a transient flow of blood. The method further comprises performing a numerical simulation of the transient flow of blood, for the same physical and boundary conditions, for the personalized model and the reconstructed model for an increasing blood flow rate that increases from a laminar flow to a developed turbulent flow, the simulation comprising, determined during the pre-simulation, initial conditions of blood flow; performing a numerical simulation of the steady flow of blood, for the same physical and boundary conditions, for the personalized model and the reconstructed model of transitional or turbulent flow, the simulation comprising, determined during the pre-simulation, initial conditions of blood flow; calculating, a blood flow energy $E_f$ for the personalized model and for the reconstructed model, for at least one of every time step of the transient flow or for fixed values of the inlet pressure and the outlet flow rate conditions, wherein the blood flow energy $E_f$ is defined as the product of the total pressure and the mass flow rate:

$$Ef = \left(\frac{1}{2}u^2 + \frac{P}{\rho}\right)Q = P_{total}Q$$

wherein ½ $u^2$ is a dynamic pressure, P/$\rho$ is a static pressure, $P_{total}$ is total pressure and Q is the flow rate through a surface perpendicular to an axis of the blood vessel; and determining absolute or relative indexes of blood flow as a function that compares at least on of pressure, velocity or energy flow between the personalized model and reconstructed model for at least one of a steady flow or a transient flow.

The method may comprise generating the reconstructed model based on the personalized model, by eliminating stenoses existing in the personalized model, resulting from atherosclerosis lesions, by numerical modification of the blood vessel geometry in areas of said stenoses to obtain a hypothetically healthy model as the reconstructed model prior to the onset of the lesions.

The method may further comprise determining a relative fractional flow reserve index ($FFR_{VCAST}$), from curve slope of function of average pressure of personalized model and average pressure of reconstructed model $P_{sten}(P_{rec})$, for a linearly increased flow rate, in each time step of transient flow simulation;

The method may further comprise determining a flow energy reference index (EFR), from a curve slope of function of an average total pressure of the personalized model and an average total pressure of the reconstructed model $P_{total\ sten}(P_{total\ rec})$, for a linearly increased flow rate, in each time step of the transient flow simulation;

The method may further comprise determining a relative fractional flow reserve index ($FFR_{VCAST}$), as a ratio of an average pressure $(P)_{sten}$ measured in the personalized model to the average pressure $(P)_{rec}$ in the reconstructed model, for the steady flow, under fixed value of the inlet pressure and the outlet flow rate conditions as:

$$FFR_{VCAST} = \frac{(P)_{sten}}{(P)_{rec}}$$

The method may further comprise determining a reference flow energy reference index (EFR) as a ratio of an average total pressure $(P_{total})_{sten}$ measured in the personalized model to the average pressure $(P_{total})_{rec}$ in the reconstructed model, for a steady flow, under a fixed value of the inlet pressure and the outlet flow rate conditions

$$EFR = \frac{(P_{total})_{sten}}{(P_{total})_{rec}}$$

The method may further comprise, taking into account zero-flow pressure ($P_0$), determining a relative Fractional Flow Reserve ($FFR_{VCAST}$) index and a Energy Flow Reference (EFR) index as:

$$FFR_{VCAST} = \frac{(P1_d)_{sten} - P_0}{(P1_d)_{rec} - P_0}$$

$$EFR = \frac{\left(P1_{total\_d}\right)_{sten} - P_0}{\left(P1_{total\_d}\right)_{rec} - P_0}$$

The method may further comprise determining a coronary inlet flow rate for the steady flow test as:

$$Qin = \beta D^n$$

wherein: β - correlation coefficient

D- diameter of inlet coronary artery

n- power number, in our example n=2.

The method may further comprise determining at least one other absolute index or relative index of hemodynamics parameters, including a vascular resistance (R), a pressure drop (dP), a turbulence kinetic energy (TKE), a wall shear stress (WSS), an oscillatory shear (OSI) and a residence time (RRT), as a function of a pressure (stress) and a flow rate of the compared personalized model and the reference model.

Another object of the invention is a computer-implemented system, comprising: at least one nontransitory processor-readable storage medium that stores at least one of processor-executable instructions or data; and at least one processor communicably coupled to at least one nontransitory processor-readable storage medium, wherein at least one processor is configured to perform the steps of the method of any of the previous claims.

BRIEF DESCRIPTION OF FIGURES

Fig. 1 shows coronary perfusion pressure-flow relationship in the presence of stenosis from rest conditions to high physical exertion conditions. Due to the regulation of coronary vascular resistance (vasodilation) blood flow is maintained relatively constant over a wide range of perfusion pressures. Outside this range, flow become pressure-dependent and the loss of autoregulation is observed. The S1 curve represents a stenotic vessel with a lesser degree of narrowing compared to S2, under maximum vasodilation conditions. The point P reflect the hypothetical state of patient healthy vessels without stenosis. Points P1 and P2 reflect the actual state of coronary vessels of the patient with existing atherosclerotic lesion. For normal coronary vessel, without stenosis, under maximal vasodilation, the loss of flow energy is associated mainly with viscous forces

Fig. 2 shows, in a simplified block diagram, the methods of modelling blood vessels and blood flow in these blood vessels models;

Fig. 3 shows a comparison of a three-dimensional personalized model with a reconstructed (i.e. reference) model of the blood vessels;

Fig. 4 shows a schematic view of a personalized model with atherosclerosis plaques as well as the reconstructed model after numerical reconstruction of the blood vessel at the site of the stenosis;

Fig. 5 shows a personalized model with visible velocity streamlines and marked flow disturbances;

Fig. 6 shows a reconstructed model with visible velocity streamlines and marked flow disturbances;

Fig.7. shows example of a virtual exercise test under a linear increase in flow rate at the inlet of the left coronary artery with 72% stenosis in mLAD (mid Left Anterior Descending Artery): a) pressure-flow function graph showing the changes in pressure on inlet (dot line), in selected branch with narrowing and the same branch of the reconstructed model, b) the relationship between pressure downstream narrowing of the personalized model (Psten) and pressure in the same place of the coronary branch of the reconstructed model (Prec). Point P0 corresponds to a pressure value for a flow rate of 0 ml /s and point P1 corresponds to a pressure value calculated from morphometric-flow-mass relationships.

Fig.8. shows example of EFR results that defined as the ratio of total flow energy losses in the branch of the stenotic model to the flow energy losses in the branch of the virtually reconstructed coronary vessel without atherosclerotic changes, for the left coronary artery with 72% stenosis in mLAD.

Fig. 9 shows the structure of a computer system for implementing the methods presented herein.

DETAILED DESCRIPTION OF THE INVENTION

The following detailed description is of the best currently contemplated modes of carrying out the invention. The description is not to be taken in a limiting sense, but is made merely for the purpose of illustrating the general principles of the invention.

The method of modelling blood vessels and blood flow in these blood vessels models allows to determine various information on blood flow, using for this purpose only non-invasively collected patient information. The modelling method may refer to blood flow in any blood vessels, such as coronary, cervical, peripheral vessels, abdominal cavities, kidneys and cerebral vessels.

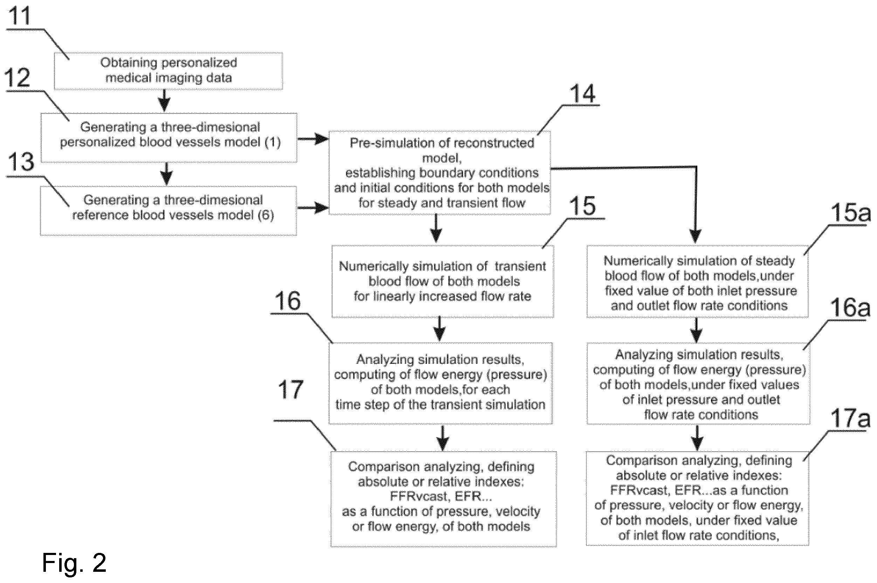

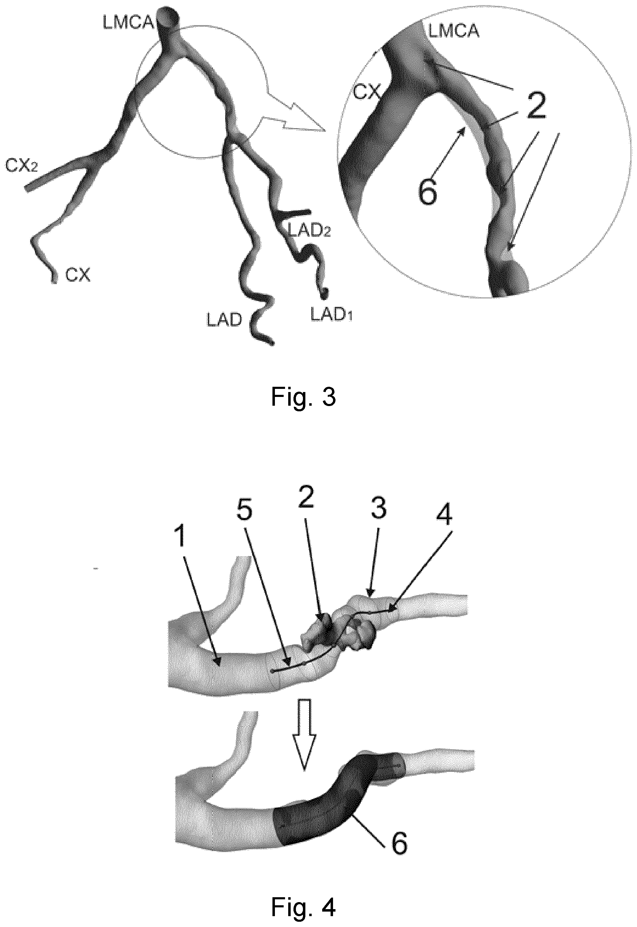

Fig. 2 shows, in a simplified block diagram, the method of modelling blood vessels and blood flow in these blood vessels models. In step 12, a personalized three-dimensional model 1 of blood vessels (as shown in Fig. 3) is generated, in this particular case it is the patient's coronary vessels model. The personalized model is generated based on the segmentation 3, 4 (as shown in Fig. 4) of the results of medical imaging examinations received in step 11. For this purpose, the available tomography scan results are used. Alternatively, the magnetic resonance test results could be used for this purpose. The coronary vessel system comprises a complex vessels network, from large arteries to arterioles, capillaries and veins. The exemplary personalized model 1, includes the *Left Main Coronary Artery* LMCA, the *Left Anterior Descending Artery* LAD with *distal branches* LAD1, LAD2, the left circumferential artery CX together with the *marginal branch* CX2.

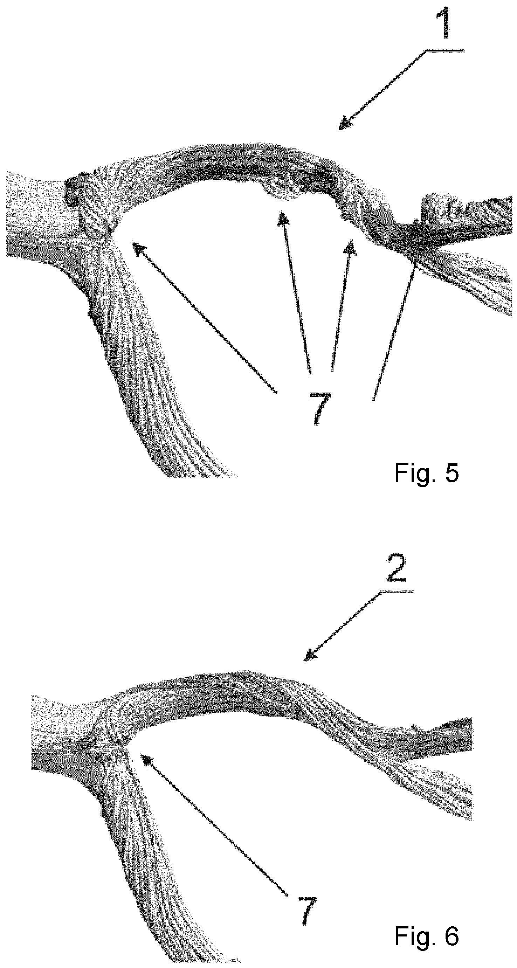

Subsequently, a three-dimensional reconstructed model 6 of blood vessels (as shown in Fig. 3 and 4), that reflects the condition of healthy blood vessels and lacks lesions 2 visible in the results of medical imaging - in tomography results, is generated in step 13 on the basis of the personalized model 1. In the personalized model 1, existing lesions 2 in the form of stenoses that induced disturbance of flow 7 (as shown in Fig. 5), are removed by numerically modifying the geometry of the blood vessel at the sites of these stenoses in order to obtain a reconstructed model of a hypothetically healthy blood vessel prior to the onset of lesions. The numerical modification of the geometry of the blood vessel is made on the basis of the approximation of the cross-sectional area in front of and behind the stenoses and curvature of the center line 5 of the vessel. In order to modify the geometry of the vessel, it is also possible to take into account the Gauss curvature of its surface. The numerical modification of the geometry of the blood vessels itself may be performed by means of any three-dimensional graphics software known in the art.

After the personalized model 1 and the reconstructed model 6 are generated, in both these models 1 and 6, (Fig.4) a volumetric calculation grid is generated in step 14 (Fig.2), for which the equation of blood flow dynamics is solved in each node to determine local velocity and pressure values, which are used at a later step of comparing the generated models 1, 6 of blood vessels between each other.

The preparing the numerical model and establishing initial boundary condition, both for steady end transient flow, is performed in step 14, based on the pre-simulation of reconstructed (non-stenotic) models, under high-intensity physical exercise conditions. The definition of these conditions is explained below.

The Doge et al. in "Lumen diameter of normal human coronary arteries. Influence of age, sex, anatomic variation, and left ventricular hypertrophy or dilation." Circulation. 86:232-246. 1992, presented a reconstructed normal data set against which to compare lumen dimensions in various pathological states. The authors says that the precise knowledge of the expected "normal" lumen diameter at a given coronary anatomic location may have the particular value in the investigation of estimate of coronary disease severity.

Based on this work it can be assumed that the maximal flow under high effort will be about 5 times higher than the average flow in the coronary artery statistically average diameter of 4.5 mm measured in the middle left main artery of right coronary artery-dominant in normal men, under resting condition.

The determine of patient specific flow rate can be estimated based on flow-mass relationships obtained from morphometric studies, presented by Choy JS in "Scaling of Myocardial Mass to Flow and Morphometry of Coronary Arteries", J Appl Physiol. 104(5): 1281-1286. 2008.

According to this studies the maximal coronary inlet flow rate can be written as:

$$Qin = \beta D^n$$

wherein: β - correlation coefficient

D- diameter of inlet coronary artery
n- power number, in our example n=2.

In the next step, numerical simulation of transient blood flow is performed in step 15 for linearly increased flow rate and pre-established identical initial conditions in both generated models 1, 6 of blood vessels, wherein during the flow simulation, the blood flow conditions are established and regulated on the inlet to the models of the blood vessels. Based on pressure and mass flow calculations, in the step 15, simultaneously on the inlet and on every outlet of these models the energy of blood flow is calculated for every time step of transient flow. Depending on necessity, the blood flow conditions on the inlet to the models may vary significantly between individual tests. The numerical flow simulation is carried out for systolic pressure and inlet flow rate with values corresponding to high exertion conditions (as described by Wolthuis RA et al, in "The response of healthy men to treadmill exercise". Circulation. 55: 153-157. 1977 and Fletcher GF et al, in "Exercise Standards for Testing and Training. A Statement for Healthcare Professionals From the American Heart Association". Circulation. 104:1694-1740. 2001). According to these data an average systolic pressure of test can be determined over a wide pressure range under high exertion conditions (P1 on the Fig. 1,7).

In step 15a, in compare to the simulation performed in step 15, the simulation may be performed for a steady flow under fixed value of inlet pressure and outlets flow rate conditions.

Using numerical calculations for steady flow instead calculations for transient flow will shorten the time needed to obtain test results from several hours to several minutes. It is extremely important from the point of view of the diagnosis of coronary vessels and the quick selection of the treatment procedure.

Conducting such simulation for steady flow, for values (inlet flow rate and systolic pressure) of P1, it is possible to spot an additional change in energy between the examined models 1, 6 of the blood vessels, which is associated with turbulence and flow separation.

Predetermined physical and boundary conditions for which blood flow in blood vessels is simulated in steps 15 and 15a, and for which flow energy is calculated in steps 16 and 16a allow easy comparison of the obtained results between the personalized model 1 and the reconstructed model 6 in steps 17 and 17a for transient and steady flow, respectively.

It allows to determine, among others, the relative energy flow reference index EFR (4), by dividing the flow energy in the branch of the personalized model 1 by the flow energy in the same branch of the reconstructed model 6, namely:

$$EFR = \frac{\left(\frac{EF_d}{EF_a}\right)_{sten}}{\left(\frac{EF_d}{EF_a}\right)_{rec}} = \frac{(EF_d)_{sten}}{(EF_d)_{rec}} = \frac{(P_{total\_d}*Q_d)_{sten}}{(P_{total\_d}*Q_d)_{rec}} = \frac{(P_{total\_d})_{sten}}{(P_{total\_d})_{rec}} \tag{1}$$

In case of loss of autoregulation, the narrowing of the vessel increases the flow resistance and causes an additional energy drop. The higher the flow rate, the higher the energy drop. During flow through stenosis, when the transition from laminar to turbulent flow occurs, the local energy losses are caused mainly by flow disturbance such as separations, secondary flow, and fluctuation of velocity and vortices.

Total energy losses during flow through the coronary vessel can be estimated as the energy difference between inlet of the vessel and the outlet from all branches (as discussed by RyuU K. TM. et al in "Importance of Accurate Geometry in the Study of the Total Cavopulmonary Connection: Computational Simulations and In Vitro Experiments", Annals of Biomedical Engineering, Vol. 29, pp. 844-853, 2001).

The mechanical energy flow rate Ef (per unit mass), expressed in Watt, which is needed to overcome the (constant) flow resistance and maintain the specified blood flow through cross sectional area of the coronary artery is represented as the product of the total pressure $P_{total}$ and the mass flow rate Q (Schobeiri, 2010) (assuming no gravitational effects):

$$Ef_i = \left(\frac{1}{2}u^2 + \frac{P}{\rho}\right)_i Q_i = (P_{total})_i \, Q_i \, [W] \tag{2}$$

In order to compare the energy loss in individual branches of the stenotic coronary vessel with the energy loss in the branches of a numerically reconstructed coronary vessel without stenosis, the relative Fractional Flow Reserve (FFR$_{VCAST}$) and Energy Flow Reference (EFR) indexes is determined.

These indices expresses, the ratio of the flow energy losses in the any branch of the personalized model to the flow energy losses in the branch of the virtually reconstructed normal coronary vessel without atherosclerotic changes.

Assuming the same inlet aortic flow energy (EF$_a$) and the same flow rate distribution (Q$_i$) at the outlet, for both the reconstructed (i.e. reference) model and the personalized (stenotic) model;

$$(EF_a)_{rec} = (EF_a)_{sten} = EF_a$$

$$(Q_i)_{rec} = (Q_i)_{sten} = Q_i$$

the relative Fractional Flow Reserve as well the Energy Flow Reference indices can be defined as:

$$FFR_{VCAST} = \frac{FFR_{sten}}{FFR_{rec}} = \frac{\left(\frac{P_d}{P_a}\right)_{sten}}{\left(\frac{P_d}{P_a}\right)_{rec}} = \frac{(P_d)_{sten}}{(P_d)_{rec}} \tag{3}$$

$$EFR = \frac{\left(\frac{EF_d}{EF_a}\right)_{sten}}{\left(\frac{EF_d}{EF_a}\right)_{rec}} = \frac{(EF_d)_{sten}}{(EF_d)_{rec}} = \frac{(P_{total\_d}*Q_d)_{sten}}{(P_{total\_d}*Q_d)_{rec}} = \frac{(P_{total\_d})_{sten}}{(P_{total\_d})_{rec}} \qquad (4)$$

where: $FFR_{sen}$ and $FFR_{rec}$ - fractional flow reserve, for both personalized and reconstructed models, determined as the ratio of the average pressure measured after the stenosis ($P_d$) in the distal section of the vessel to the average pressure in the aorta ($P_a$) in the conditions of maximum hyperaemia, $\left(\frac{EF_d}{EF_a}\right)$ - energy drop for both personalized (sten) and reconstructed (rec) models.

Based on the equation 4, it can be concluded that the indices EFR, can be calculated as the ratio of the distal total pressure in branch of the personalized model to the distal total pressure in branch of reconstructed model.

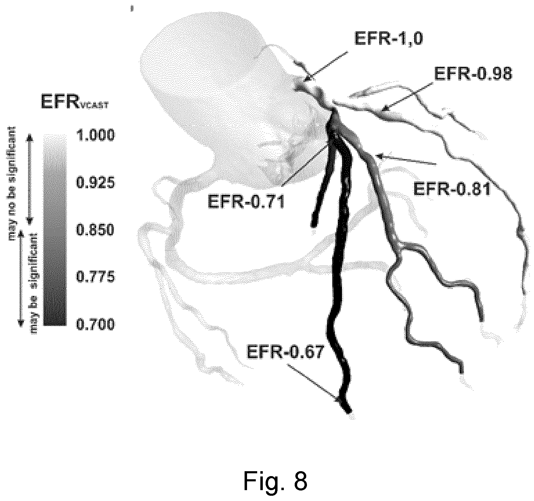

For normal arteries, without stenosis, these indices are close to 1. For all branches, where stenosis has an effect on flow, this parameter take a value between 1 and 0, see Fig.8. The greater the impact of stenosis in the branch on flow energy drop in any branch, the lower the values of the indices.

The pre-simulation of reconstructed (non-stenotic) models, in exercise conditions, enabled the inventors to determine the correlation between the resistance of downstream vasculature of each coronary branch ($R_i$), outflow $Q_i$ and pressure $P_i$ of the coronary tree. Taking into account zero-flow pressure $P_0$, i.e. the arterial blood pressure level at which flow stops (as discussed by Spaan JAE et al. In "Physiological basis of clinically used coronary hemodynamic indices", Circulation. 113; 446-455. 2006) the resistance Ri can be defined as:

$$Ri = \frac{P_i - P_0}{Q_i} \qquad (6)$$

In earlier publications (Dole WP et al in "Diastolic coronary artery pressure-flow velocity relationships in conscious man", Cardiovasc Res. 18(9): 548-554. 1984 and Nanto S et al in "Determination of coronary zero flow pressure by analysis of the baseline pressure - flow relationship in humans" Jpn Circ J. 65 (9): 793-796. 2001) the $P_0$ value, in these study was determined at 5 kPa.

Running a full, transient (time-dependent) flow, test during a linearly increasing inlet flow rate up to achieve of conditions of maximum physical exertion is very time consuming. However, Lo et al in "On outflow boundary conditions for CT-based computation of FFR: Examination using PET images", Med Eng Phys. 76:79-87. doi: 10.1016/j.medengphy.2019.10.007. 2020), showed high correlation between FFR values obtained using a pulsatile simulation and that of a steady-state simulation. Based on the findings of this work, the maximum discrepancy in numerical computed FFR from steady and unsteady flow simulations is 2 %.

Therefore, finally, some indexes discloses in EP3629341A1, such as $FFR_{VCAST}$, the reference fractional flow reserve index, and EFR, the energy flow reference index, can be calculated for steady flow under the conditions of maximum physical effort. The inventors found that this will significantly reduce the numerical computation time.

To test the assumptions of the present invention, for the acceleration of the execution time of test, a high-performance computer (HPC) was used.

Parallel processing was performed using 120 CPUs. Average wall-clock time for typical steady flow test of coronary arteries was approximately 540s whereas for the transient flow, this time was over 100 times higher, mainly because of the greater number of time steps for transient flow. In this case the calculation was performed with total time of 2s and time step of 0.002s.

Using numerical calculations for steady flow instead calculations for transient flow will shorten the time needed to obtain test results from several hours to several minutes. It is extremely important from the point of view of the diagnosis of coronary vessels and the quick selection of the treatment procedure.

The invention is based on the comparative method therefore it makes possible to determine the relationship between pressure in the model with stenosis and the pressure in the reconstructed model under the same hemodynamic conditions. Our own research showed a high linear correlation, see Fig.7, between distal pressure, downstream to the stenosis and the pressure in the same place in the reconstructed model, during linear increase of flow rate. For this reason the angle of the curve slope can be a new parameter that corresponds to the haemodynamic significance of vessel stenosis.

For high flow rate (high-intensity physical exercise conditions) this angle is independent of the flow rate (see Fig 7).

The constant slope of the curve makes it possible to determine of parameters related to the coronary flow, for the steady-flow, at any point in this curve.

Taking into account zero-flow pressure and assuming that the relationship Psten (Prec) is linear (as seen in Fig. 1 and 7), equations 3 and 4 can be written as:

$$FFR_{VCAST} = \frac{(P1_d)_{sten} - P_0}{(P_{1d})_{rec} - P_0} \tag{7}$$

$$EFR = \frac{(P1_{total\_d})_{sten} - P_0}{(P1_{total\_d})_{rec} - P_0} \tag{8}$$

where $P1_d$ is the pressure, downstream to stenosis, calculated for both models (sten, rec) under high effort conditions.

Based on the comparison of the simulation results of the personalized model (1) and a reference model (6), also other hemodynamic parameters, which depended on the size of flow disturbances caused by vessel stenosis, can be specified by a functions of pressure (stress) and of flow rate.

Among them, we can distinguish absolute and relative indexes of: vascular resistance R, turbulent kinetic energy, *TKE* pressure drop *DP,* wall shear stress *WSS,* shear oscillators *OSI* or residence time *RRT.*

The flow disorders are closely related to turbulent kinetic energy (TKE). For turbulent flow, the velocity can be represented as the sum of the mean value and fluctuation component and the kinetic energy of turbulence can be specified as the root mean square velocity fluctuations:

$$TKE = \frac{1}{2}\overline{\rho u_j' u_j'} = \frac{1}{2}\rho\left(\overline{u_1'^2} + \overline{u_2'^2} + \overline{u_3'^2}\right) [mJ\ kg^{-1}] \tag{9}$$

where $u_j'$ is the fluctuation of the *j*-th velocity component.

The wall shear stress (*WSS*) is the tangential frictional force on the endothelial surface. For a Newtonian fluid flow in the straight vessel the shear stress is proportional to the flow shear and can be expressed as:

$$WSS = \mu \frac{\partial u}{\partial n}\Big|_{wall} \tag{10}$$

where m is the dynamic viscosity, u is the flow velocity parallel to the wall.

Additionally, in the case of pulsating flow the oscillatory shear index *OSI* and residence time RRT indexes, based on the temporal fluctuation of the WSS, may be computed respectively as follows:

$$OSI = \frac{1}{2}\left(1 - \frac{\left|\int_0^T \overline{WSS}dt\right|}{\int_0^T |\overline{WSS}|dt}\right) \tag{11}$$

$$RRT = \left[(1 - 2OSI)\frac{1}{T}\int_0^T |\overline{WSS}|\ dt\right]^{-1} \tag{12}$$

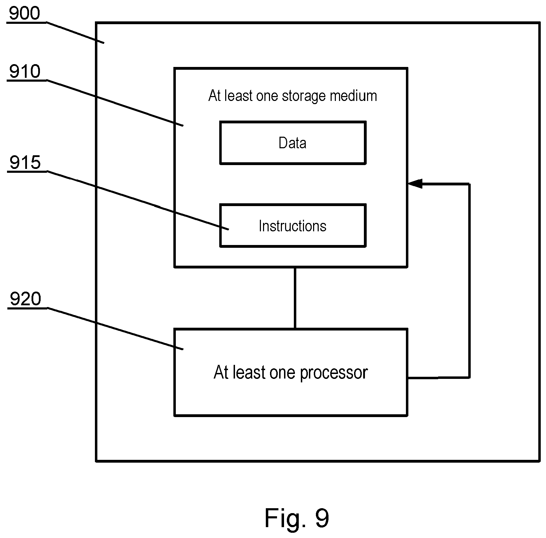

The functionality described herein can be implemented in a computer system 900, such as shown in Fig. 9. The system 900 may include at least one nontransitory processor-readable storage medium 910 that stores at least one of processor-executable instructions 915 or data; and at least one processor 920 communicably coupled to the at least one nontransitory processor-readable storage medium 910. The at least one processor 920 may be configured to (by executing the instructions 915) perform the methods presented herein, in particular the method presented in Fig. 2.

The claimed invention as recited in the claims that follow is not limited to the embodiments described herein.

## Claims

1. A computer-implemented method for modelling blood vessels, the method comprising the steps of:

   - obtaining (11) medical imaging data of the blood vessels;
   - generating (12) a three-dimensional personalized model (1) of the blood vessels, based on the medical imaging data;
   - generating (13) a three-dimensional reconstructed model (6) of the blood vessels that reflects a state of healthy blood vessels that lack lesions, based on the medical imaging data or based on numerical reconstruction of the personalized model;
- performing pre-simulation (14) of reconstructed model, establishing boundary conditions and initial conditions for both models for a steady flow of blood and a transient flow of blood;

**characterized by**:

- performing (15) a numerical simulation of the transient flow of blood, for the same physical and boundary conditions, for the personalized model (1) and the reconstructed model (6) for an increasing blood flow rate that increases from a laminar flow to a developed turbulent flow, the simulation comprising, determined during the pre-simulation (14), initial conditions of blood flow;
- performing (15a) a numerical simulation of the steady flow of blood, for the same physical and boundary conditions, for the personalized model (1) and the reconstructed model (6) of transitional or turbulent flow, the simulation comprising, determined during the pre-simulation (14), initial conditions of blood flow;
- calculating (16), (16a) a blood flow energy $E_f$ for the personalized model (1) and for the reconstructed model (6), for at least one of every time step of the transient flow or for fixed values of the inlet pressure and the outlet flow rate conditions, wherein the blood flow energy $E_f$ is defined as the product of the total pressure and the mass flow rate:

$$Ef = \left(\frac{1}{2}u^2 + \frac{P}{\rho}\right)Q = P_{total}Q$$

wherein $\frac{1}{2}u^2$ is a dynamic pressure, $P/\rho$ is a static pressure, $P_{total}$ is total pressure and Q is the flow rate through a surface perpendicular to an axis of the blood vessel; and
- determining (17,17a) absolute or relative indexes of blood flow as a function that compares at least on of pressure, velocity or energy flow between the personalized model (1) and reconstructed model (6) for at least one of a steady flow or a transient flow.

2. The method according to claim 1, comprising generating the reconstructed model based on the personalized model, by eliminating stenoses existing in the personalized model, resulting from atherosclerosis lesions, by numerical modification of the blood vessel geometry in areas of said stenoses to obtain a hypothetically healthy model as the reconstructed model prior to the onset of the lesions.

3. The method according to any of previous claims, further comprising determining a relative fractional flow reserve index (FFR$_{VCAST}$), from curve slope of function of average pressure of personalized model and average pressure of reconstructed model $P_{sten}(P_{rec})$, for a linearly increased flow rate, in each time step of transient flow simulation;

4. The method according to any of previous claims, further comprising determining a flow energy reference index (EFR), from a curve slope of function of an average total pressure of the personalized model and an average total pressure of the reconstructed model $P_{total\ sten}(P_{total\ rec})$, for a linearly increased flow rate, in each time step of the transient flow simulation;

5. The method according to any of previous claims, further comprising determining a relative fractional flow reserve index (FFR$_{VCAST}$), as a ratio of an average pressure $(P)_{sten}$ measured in the personalized model (1) to the average pressure $(P)_{rec}$ in the reconstructed model (6), for the steady flow, under fixed value of the inlet pressure and the outlet flow rate conditions as:

$$FFR_{VCAST} = \frac{(P)_{sten}}{(P)_{rec}}$$

6. The method according to any of previous claims, further comprising determining a reference flow energy reference index (EFR) as a ratio of an average total pressure $(P_{total})_{sten}$ measured in the personalized model (1) to the average pressure $(P_{total})_{rec}$ in the reconstructed model(6), for a steady flow, under a fixed value of the inlet pressure and the outlet flow rate conditions

$$EFR = \frac{(P_{total})_{sten}}{(P_{total})_{rec}}$$

7. The method according to any of previous claims, further comprising, taking into account zero-flow pressure ($P_0$), determining a relative Fractional Flow Reserve ($FFR_{VCAST}$) index and a Energy Flow Reference (EFR) index as:

$$FFR_{VCAST} = \frac{(P1_d)_{sten} - P_0}{(P_{1d})_{rec} - P_0}$$

$$EFR = \frac{(P1_{total\_d})_{sten} - P_0}{(P1_{total\_d})_{rec} - P_0}$$

8. The method according to any of previous claims, further comprising determining a coronary inlet flow rate for the steady flow test as:

$$Qin = \beta D^n$$

wherein: $\beta$ - correlation coefficient

D- diameter of inlet coronary artery
n- power number, in our example n=2.

9. The method according to claim 1, comprising determining at least one other absolute index or relative index of hemodynamics parameters, including a vascular resistance (R), a pressure drop (dP), a turbulence kinetic energy (TKE), a wall shear stress (WSS), an oscillatory shear (OSI) and a residence time (RRT), as a function of a pressure (stress) and a flow rate of the compared personalized model (1) and the reference model (6).

10. A computer-implemented system, comprising:

- at least one nontransitory processor-readable storage medium (910) that stores at least one of processor-executable instructions or data; and
- at least one processor (920) communicably coupled to at least one nontransitory processor-readable storage medium, wherein at least one processor is configured to perform the steps of the method of any of the previous claims.

**Patentansprüche**

1. Computerimplementiertes Verfahren zum Modellieren von Blutgefäßen, wobei das Verfahren die folgenden Schritte umfasst:

- Erhalten (11) von medizinischen Bildgebungsdaten der Blutgefäße;
- Erzeugen (12) eines dreidimensionalen personalisierten Modells (1) der Blutgefäße basierend auf den medizinischen Bildgebungsdaten;
- Erzeugen (13) eines dreidimensionalen rekonstruierten Modells (6) der Blutgefäße, das einen Zustand von gesunden Blutgefäßen ohne Läsionen wiedergibt, basierend auf den medizinischen Bildgebungsdaten oder basierend auf numerischer Rekonstruktion des personalisierten Modells;
- Durchführen einer Vorsimulation (14) eines rekonstruierten Modells, wobei Randbedingungen und Anfangsbedingungen sowohl für ein Modell für einen gleichmäßigen Blutfluss als auch für einen schwankenden Blutfluss festgelegt werden;

**gekennzeichnet durch**:

- Durchführen (15) einer numerischen Simulation des schwankenden Blutflusses für die gleichen physikalischen und Randbedingungen für das personalisierte Modell (1) und das rekonstruierte Modell (6) für eine ansteigende Blutflussrate, die von einem laminaren Fluss zu einem entwickelten turbulenten Fluss ansteigt, wobei die Simulation, bestimmt während der Vorsimulation (14), Anfangsbedingungen von Blutfluss umfasst;

- Durchführen (15a) einer numerischen Simulation des gleichmäßigen Blutflusses für die gleichen physikalischen und Randbedingungen für das personalisierte Modell (1) und das rekonstruierte Modell (6) von Übergangs- oder turbulenter Strömung, wobei die Simulation, bestimmt während der Vorsimulation (14), Anfangsbedingungen von Blutfluss umfasst;

- Berechnen (16), (16a) einer Blutflussenergie $E_f$ für das personalisierte Modell (1) und für das rekonstruierte Modell (6), für zumindest einen von jedem Zeitschritt des schwankenden Flusses oder für feste Werte der Einlassdruck- und der Auslassflussratenbedingungen, wobei die Blutflussenergie $E_f$ als das Produkt des Gesamtdrucks und der Massenflussrate definiert ist:

$$Ef = \left(\frac{1}{2}u^2 + \frac{P}{\rho}\right)Q = P_iP_{total}Q$$

,

wobei $\frac{1}{2}u^2$ ein dynamischer Druck ist, $P/\rho$ ein statischer Druck ist, $P_{total}$ der Gesamtdruck ist und Q die Flussrate durch eine Oberfläche senkrecht zu einer Achse des Blutgefäßes ist; und

- Bestimmen (17,17a) von absoluten oder relativen Indizes von Blutfluss als eine Funktion, die zumindest eines von Druck, Geschwindigkeit oder Energiefluss zwischen dem personalisierten Modell (1) und dem rekonstruierten Modell (6) für zumindest eines von einem gleichmäßigen Fluss oder einem schwankenden Fluss vergleicht.

2. Verfahren nach Anspruch 1, umfassend Erzeugen des rekonstruierten Modells basierend auf dem personalisierten Modell durch Eliminieren von Stenosen, die in dem personalisierten Modell existieren, die aus Atheroskleroseläsionen resultieren, durch numerische Modifikation der Blutgefäßgeometrie in Bereichen der Stenosen, um ein hypothetisch gesundes Modell als das rekonstruierte Modell vor dem Einsetzen der Läsionen zu erhalten.

3. Verfahren nach einem der vorhergehenden Ansprüche, ferner umfassend Bestimmen eines relativen fraktionalen Fluss-Reserveindex ($FFR_{VCAST}$), aus einer Kurvensteigung einer Funktion eines durchschnittlichen Drucks eines personalisierten Modells und eines durchschnittlichen Drucks eines rekonstruierten Modells $P_{sten}(P_{rec})$ für eine linear gesteigerte Flussrate in jedem Zeitschritt einer schwankenden Flusssimulation.

4. Verfahren nach einem der vorhergehenden Ansprüche, ferner umfassend Bestimmen eines Flussenergie-Referenzindex (EFR) aus einer Kurvensteigung einer Funktion eines durchschnittlichen Gesamtdrucks des personalisierten Modells und eines durchschnittlichen Gesamtdrucks des rekonstruierten Modells $P_{total\ sten}(P_{total\ rec})$ für eine linear gesteigerte Flussrate in jedem Zeitschritt der schwankenden Flusssimulation.

5. Verfahren nach einem der vorhergehenden Ansprüche, ferner umfassend Bestimmen eines relativen fraktionalen Fluss-Reserve-Index ($FFR_{VCAST}$) als ein Verhältnis eines durchschnittlichen Drucks $(P)_{sten}$, gemessen in dem personalisierten Modell (1), zu dem durchschnittlichen Druck $(P)_{rec}$ in dem rekonstruierten Modell (6), für den gleichmäßigen Fluss, unter einem festen Wert der Einlassdruck- und der Auslassflussratenbedingungen als:

$$FFR_{VCAST} = \frac{(P)_{sten}}{(P)_{rec}}$$

.

6. Verfahren nach einem der vorhergehenden Ansprüche, ferner umfassend Bestimmen eines Referenzflussenergie-Referenzindex (EFR) als ein Verhältnis eines durchschnittlichen Gesamtdrucks $(P_{total})_{sten}$, gemessen in dem personalisierten Modell (1), zu dem durchschnittlichen Druck $(P_{total})_{rec}$ in dem rekonstruierten Modell (6), für einen gleichmäßigen Fluss, unter einem festen Wert der Einlassdruck- und der Auslassflussratenbedingungen

$$EFR = \frac{(P_{total})_{sten}}{(P_{total})_{rec}}$$

.

7. Verfahren nach einem der vorhergehenden Ansprüche, ferner umfassend, unter Berücksichtigung von Nullflussdruck

($P_0$), Bestimmen eines relativen fraktionalen Flussreserve(FFR$_{VCAST}$-)Index und eines Energieflussreferenz(EFR-) Index als:

$$FFR_{VCAST} = \frac{(P1_d)_{sten} - P_0}{(P_{1d})_{rec} - P_0}$$

$$EFR = \frac{(P1_{total\_d})_{sten} - P_0}{(P1_{total\_d})_{rec} - P_0} .$$

8. Verfahren nach einem der vorhergehenden Ansprüche, ferner umfassend Bestimmen einer Koronareinlassflussrate für den gleichmäßigen Flusstest als:

$$Qin = \beta D^n ,$$

wobei: $\beta$ - Korrelationskoeffizient,

D- Durchmesser von Einlasskoronararterie,
n- Potenzzahl, in unserem Beispiel n=2.

9. Verfahren nach Anspruch 1, umfassend Bestimmen von zumindest einem anderen absoluten Index oder relativen Index von Hämodynamikparametern, einschließlich einem Gefäßwiderstand (R), einem Druckabfall (dP), einer kinetischen Turbulenzenergie (TKE), einer Wandschubspannung (WSS), einer oszillatorischen Scherung (OSI) und einer Verweilzeit (RRT), als eine Funktion eines Drucks (Spannung) und einer Flussrate des verglichenen personalisierten Modells (1) und des Referenzmodells (6).

10. Computerimplementiertes System, umfassend:

- zumindest ein nichtflüchtiges prozessorlesbares Speichermedium (910), das zumindest eines von prozessorausführbaren Anweisungen oder Daten speichert; und
- zumindest einen Prozessor (920), der kommunizierbar an zumindest ein nichtflüchtiges prozessorlesbares Speichermedium gekoppelt ist, wobei zumindest ein Prozessor konfiguriert ist, um die Schritte des Verfahrens nach einem der vorhergehenden Ansprüche durchzuführen.

**Revendications**

1. Procédé mis en œuvre par ordinateur de modélisation des vaisseaux sanguins, le procédé comprenant les étapes de :

- obtention (11) de données d'imagerie médicale des vaisseaux sanguins ;
- génération (12) d'un modèle tridimensionnel personnalisé (1) des vaisseaux sanguins, sur la base des données d'imagerie médicale ;
- génération (13) d'un modèle tridimensionnel reconstruit (6) des vaisseaux sanguins qui reflète un état des vaisseaux sanguins sains qui sont dépourvus de lésions, sur la base des données d'imagerie médicale ou sur la base de la reconstruction numérique du modèle personnalisé ;
- réalisation d'une pré-simulation (14) de modèle reconstruit, en établissant des conditions limites et des conditions initiales pour les deux modèles pour un flux sanguin constant et un flux sanguin transitoire ;

**caractérisé par** :

- la réalisation (15) d'une simulation numérique du flux sanguin transitoire, pour les mêmes conditions physiques et limites, pour le modèle personnalisé (1) et le modèle reconstruit (6) pour un débit sanguin croissant qui augmente d'un flux laminaire à un flux turbulent développé, la simulation comprenant, déterminées lors de la pré-simulation (14), des conditions initiales de flux sanguin ;
- la réalisation (15a) d'une simulation numérique du flux sanguin constant, pour les mêmes conditions physiques et limites, pour le modèle personnalisé (1) et le modèle reconstruit (6) de flux transitoire ou turbulent, la simulation comprenant, déterminées lors de la pré-simulation (14), des conditions initiales de flux sanguin ;
- le calcul (16), (16a) d'une énergie de flux sanguin $E_f$ pour le modèle personnalisé (1) et pour le modèle reconstruit (6), pour au moins un de chaque étape temporelle du flux transitoire ou pour des valeurs fixes de la pression d'entrée et des conditions de débit de sortie, dans lequel l'énergie de flux sanguin $E_f$ est définie comme le produit de la pression totale et du débit massique :

$$Ef = \left(\frac{1}{2}u^2 + \frac{P}{\rho}\right)Q = PP_{total}Q$$

où ½ $u^2$ représente une pression dynamique, $P/\rho$ représente une pression statique, $P_{total}$ représente la pression totale et Q représente le débit à travers une surface perpendiculaire à un axe du vaisseau sanguin ; et
- la détermination (17, 17a) d'indices absolus ou relatifs de flux sanguin en tant que fonction qui compare au moins la pression, la vitesse ou le flux d'énergie entre le modèle personnalisé (1) et le modèle reconstruit (6) pour au moins l'un d'un flux constant ou d'un flux transitoire.

2. Procédé selon la revendication 1, comprenant la génération du modèle reconstruit sur la base du modèle personnalisé, en éliminant les sténoses existant dans le modèle personnalisé, résultant de lésions d'athérosclérose, par modification numérique de la géométrie des vaisseaux sanguins dans les zones desdites sténoses de manière à obtenir un modèle hypothétiquement sain en tant que modèle reconstruit avant l'apparition des lésions.

3. Procédé selon l'une quelconque des revendications précédentes, comprenant en outre la détermination d'un indice de réserve de flux fractionnaire relatif ($FFR_{VCAST}$), à partir de la pente de courbe de la fonction de la pression moyenne du modèle personnalisé et de la pression moyenne du modèle reconstruit $P_{sten}(P_{rec})$, pour un débit augmenté linéairement, à chaque étape temporelle de la simulation de flux transitoire.

4. Procédé selon l'une quelconque des revendications précédentes, comprenant en outre la détermination d'un indice de référence d'énergie de flux (EFR), à partir d'une pente de courbe de la fonction d'une pression totale moyenne du modèle personnalisé et d'une pression totale moyenne du modèle reconstruit $P_{sten\,total}$ ($P_{rec\,total}$), pour un débit augmenté linéairement, à chaque étape temporelle de la simulation de flux transitoire.

5. Procédé selon l'une quelconque des revendications précédentes, comprenant en outre la détermination d'un indice de réserve de flux fractionnaire relatif ($FFR_{VCAST}$), en tant que rapport d'une pression moyenne $(P)_{sten}$ mesurée dans le modèle personnalisé (1) à la pression moyenne $(P)_{rec}$ dans le modèle reconstruit (6), pour le flux constant, sous une valeur fixe de la pression d'entrée et des conditions de débit de sortie en tant que :

$$FFR_{VCAST} = \frac{(P)_{sten}}{(P)_{rec}}$$

6. Procédé selon l'une quelconque des revendications précédentes, comprenant en outre la détermination d'un indice de référence d'énergie de flux de référence (EFR) en tant que rapport d'une pression totale moyenne $(P_{total})_{sten}$ mesurée dans le modèle personnalisé (1) à la pression moyenne $(P_{total})_{rec}$ dans le modèle reconstruit (6), pour un flux constant, sous une valeur fixe de la pression d'entrée et des conditions de débit de sortie

$$EFR = \frac{(P_{total})_{sten}}{(P_{total})_{rec}}$$

7. Procédé selon l'une quelconque des revendications précédentes, comprenant en outre, en tenant compte de la pression à flux nul ($P_0$), la détermination d'un indice relatif de réserve de flux fractionnaire ($FFR_{VCAST}$) et d'un indice de référence de flux d'énergie (EFR) en tant que :

$$FFR_{VCAST} = \frac{(P1_d)_{sten} - P_0}{(P_{1d})_{rec} - P_0}$$

$$EFR = \frac{(P1_{total\_d})_{sten} - P_0}{(P1_{total\_d})_{rec} - P_0}$$

8. Procédé selon l'une quelconque des revendications précédentes, comprenant en outre la détermination d'un débit d'entrée coronaire pour le test de flux constant en tant que :

$$Qin = \beta D^n$$

dans lequel : $\beta$ - coefficient de corrélation
D - diamètre de l'artère coronaire d'entrée
n- nombre de la puissance, dans notre exemple n=2.

9. Procédé selon la revendication 1, comprenant la détermination d'au moins un autre indice absolu ou indice relatif de paramètres hémodynamiques, comprenant une résistance vasculaire (R), une chute de pression (dP), une énergie cinétique de turbulence (TKE), une contrainte de cisaillement de paroi (WSS), un cisaillement oscillatoire (OSI) et un temps de séjour (RRT), en fonction d'une pression (contrainte) et d'un débit du modèle personnalisé comparé (1) et du modèle de référence (6).

10. Système mis en œuvre par ordinateur, comprenant :

- au moins un support de stockage non transitoire lisible par processeur (910) qui stocke au moins l'une de données ou d'instructions exécutables par processeur ; et
- au moins un processeur (920) couplé en communication à au moins un support de stockage non transitoire lisible par processeur, dans lequel au moins un processeur est configuré pour réaliser les étapes du procédé selon l'une quelconque des revendications précédentes.

Fig. 1

Fig. 2

Fig. 3

Fig. 4

Fig. 5

Fig. 6

Fig. 7

Fig. 8

900

910

915

At least one storage medium

Data

Instructions

920

At least one processor

Fig. 9

## REFERENCES CITED IN THE DESCRIPTION

### Patent documents cited in the description

- EP 2845537 A
- US 2014249790 A1
- US 2017265831 A1
- US 2014200867 A
- US 2015065846 A
- US 2012041318 A
- EP 3629341 A1
- US 9087147 B1

### Non-patent literature cited in the description

- **CRYSTAL GEORGE J**. Fractional Flow Reserve: Physiological Basis, Advantages and Limitations and Potential Gender Differences. *Current Cardiology Reviews*, 2015, vol. 11, 209-219
- **FLETCHER GF et al.** Exercise Standards for Testing and Training. A Statement for Healthcare Professionals From the American Heart Association. *Circulation.*, 2001, vol. 104, 1694-1740
- **WOLTHUIS RA et al.** The response of healthy men to treadmill exercise. *Circulation*, 1977, vol. 55, 153-157
- **MALOTA Z. et al.** Numerical analysis of the impact of flow rate, heart rate, vessel geometry, and degree of stenosis on coronary hemodynamic indices. *BMC Cardiovascular Disorders*, 2018, vol. 18, 132
- **YOUNG DF** ; **TSAI FY**. Flow characteristics in models of arterial stenoses: I Steady flow. *J Biomech*, 1973, vol. 6, 395-410
- **DUNCKER D. et al.** Regulation of Coronary Blood Flow in Health and Ischemic Heart Disease. *Prog Cardiovasc Dis.*, 2015, vol. 57 (5), 409-422
- Hemodynamic Effects of Epicardial Stenoses. **CASADONTE L.** ; **SIEBES M**. Physiological Assessment of Coronary Stenoses and the Microcirculation. Springer, 2017
- **LO et al.** On outflow boundary conditions for CT-based computation of FFR: Examination using PET images. *Med Eng Phys*, vol. 76, 79-87
- **DOGE et al.** Lumen diameter of normal human coronary arteries. Influence of age, sex, anatomic variation, and left ventricular hypertrophy or dilation.. *Circulation*, 1992, vol. 86, 232-246
- **CHOY JS**. Scaling of Myocardial Mass to Flow and Morphometry of Coronary Arteries. *J Appl Physiol.*, 2008, vol. 104 (5), 1281-1286
- **FLETCHER GF et al.** Exercise Standards for Testing and Training. A Statement for Healthcare Professionals From the American Heart Association. *Circulation*, 2001, vol. 104, 1694-1740
- **RYUU K. TM et al.** Importance of Accurate Geometry in the Study of the Total Cavopulmonary Connection: Computational Simulations and In Vitro Experiments. *Annals of Biomedical Engineering*, 2001, vol. 29, 844-853
- **SPAAN JAE et al.** Physiological basis of clinically used coronary hemodynamic indices. *Circulation*, 2006, vol. 113, 446-455
- **DOLE WP et al.** Diastolic coronary artery pressure-flow velocity relationships in conscious man. *Cardiovasc Res*, 1984, vol. 18 (9), 548-554
- **NANTO S et al.** Determination of coronary zero flow pressure by analysis of the baseline pressure - flow relationship in humans. *Jpn Circ J*, 2001, vol. 65 (9), 793-796
- **HOWEVER, LO et al.** On outflow boundary conditions for CT-based computation of FFR: Examination using PET images. *Med Eng Phys*, 2020, vol. 76, 79-87